(19) Europäisches Patentamt
European Patent Office
Office européen des brevets

(11) Publication number: **0 310 132**
**A2**

(12) # EUROPEAN PATENT APPLICATION

(21) Application number: 88116274.7

(22) Date of filing: 01.10.88

(51) Int. Cl.4: G01N 33/58 , G01N 33/577 , G01N 33/571 , //G01N33/543, G01N33/569,G01N33/576

(30) Priority: 02.10.87 US 103640

(43) Date of publication of application: 05.04.89 Bulletin 89/14

(84) Designated Contracting States: BE CH DE FR GB IT LI LU NL SE

(71) Applicant: E.I. DU PONT DE NEMOURS AND COMPANY
1007 Market Street
Wilmington Delaware 19898(US)

(72) Inventor: Bobrow, Mark Norman
3 Bonnie Way
Woburn Masssachusetts 01801(US)
Inventor: Cukor, George
7 Kensington Heights
Worcester Massachusetts 01602(US)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **Immunoassay utilizing IgG capture antibody and multiple monoclonal antibody detection system.**

(57) Immunoassays and kits for detecting or quantitating a substance having multiple epitopes which utilize IgG capture antibody and a cocktail of at least two labeled monoclonal antibodies as the detection system wherein each monoclonal antibody recognizes a distinct antigenic determinant. The labeled monoclonal antibodies may be labeled with a component of a reporter system or they may be labeled with a member of a specific immune or non-immune binding pair.

EP 0 310 132 A2

# IMMUNOASSAY UTILIZING IgG CAPTURE ANTIBODY AND MULTIPLE MONOCLONAL ANTIBODY DETECTION SYSTEM

## FIELD OF THE INVENTION

This invention relates generally to immunoassays and more specifically to solid phase immunoassays utilizing a labeled multiple monoclonal antibody detection system.

## BACKGROUND OF THE INVENTION

All immunoassays for antigens are based on the binding of specific antibody to an antigen which might be present in a specimen and the subsequent identification of the antigen-antibody complex by means of a defined detection system. The performance characteristics of immunoassay systems are dependent to a great extent on the binding characteristics of the immunoreactants utilized. Techniques allowing for the production of unlimited supplies of reagents with defined reactivities are crucial. Thus, the evolving technology related to the production and development of monoclonal antibodies has found widespread interest among investigators who use immunoassays to detect a variety of substances having multiple antigenic determinants.

Researchers recognized the potential advantages in using monoclonal antibodies for immunoassay systems. For example, a consistent supply of a well-defined reagent should allow for the wide-scale availability of immunoassay systems with defined performance characteristics. Since immunoassays follow the law of mass action the availability of immunoglobulin preparations with every molecule directed against antigen improves the kinetics and thus the sensitivity of immunoassay systems. This is particularly true of solid phase immunoassays because the concentration of immunoglobulin which can be utilized in these systems is limited by the binding properties of the solid phase.

Notwithstanding the multitude of advantages associated with the use of monoclonal antibodies in immunoassay systems, there also are a number of potential disadvantages which investigators could face in working with monoclonal antibodies. Disadvantages in the use of monoclonal antibodies to viral antigens include the possibility that the monoclonal antibodies might recognize antigenic determinants which would be present on the immunizing strains of viral antigen but not be found widely in viruses infecting humans. This might lead to false-negative reactions. Another possibility is that since monoclonals react with a specific antigenic determinant, they might be difficult to use in sandwich assays involving the formation of solid phase antibody-antigen-liquid phase antibody complexes. Monoclonal antibodies can be difficult to attach to a solid phase surface either by nonspecific absorption or covalent attachment techniques and researchers have encountered difficulty labeling monoclonals with various reporter groups using standard labeling techniques.

Although researchers have made great strides in the development and use of monoclonal antibodies, no one heretofore has developed an immunoassay using a labeled multiple monoclonal antibody detection system to detect and/or quantify a substance having multiple epitopes.

Numerous articles and patents have appeared describing a multitude of immunoassays employing monoclonal antibodies directed against a wide array of substances:

U.S. Patent 4,659,678 relates to a method of solid phase immunoassay having a reduced incubation period which is achieved by reacting a sample and antibody reagents in the liquid phase and then capturing the reaction product with an insolubilized anti-antibody generated against the unlabeled antibody reagent.

U.S. Patent 4,535,057 describes a solid phase immunoassay for detecting HSV antigens or anti-HSV IgM immunoglobulin in biological fluids by using monoclonal IgM HSV virus/antigen specific antibodies and a readably labeled, preferably biotin-avidin based, detection system.

U.S. Patent 4,430,437 describes test methods using monoclonal antibodies directed to HSV type 1 and type 2 nucleocapsid proteins.

U.S. Patent 4,271,140 describes a solid phase immunoassay using an enzyme based detection system.

U.S. Patent 4,514,505 describes radioimmunoassays with enhanced sensitivity for polypeptides (e.g. human chorionic gonadotropin) by utilizing a mixture of monoclonal antibodies.

U.S. Patent 4,228,237 describes insoluble phase enzyme immunoassays employing labeled avidin and biotin labeled reagent to detect a ligand.

U.S. Patent Re. 31,006 describes an insoluble phase immunoassay using an enzyme based detection system.

Collins, et al., J. Clinical Microbiology, Vol. 21, No. 3, pp. 375-380 (Mar. 1985) describe the development of an enzyme-linked immunosorbent assay to detect bovine herpes virus type 1 using a monclonal antibody pair, one for capture and one for detection.

Nerukar, et al., J. Clinical Microbiology, Vol. 20, No. 1, pp. 108-114 (July 1984) describe an enzyme-linked immunosorbent assay (ELISA) in which a polyclonal antibody labeled with biotin and enzyme-labeled streptavidin are used for detection.

Adler-Storthz, et al., J. Clinical Microbiology, Vol. 18, No. 6, pp. 1329-1334 (Dec. 1983) describe a biotin-avidin amplified enzyme-linked immunosorbent assay in which a biotinylated mouse monoclonal IgM antibody to HSV was used to detect HSV-1 and HSV-2.

There are also a number of articles which discuss identifying and typing herpes simplex virus:

Syva Co. Product Insert for Herpes Simplex Virus-1/Herpes Simplex Virus-2 Culture Confirmation/Typing Test (1984) describes a test to identify and type herpes simplex virus in tissue culture using multiple monoclonal antibodies labeled with fluorescein isothiocyanate.

Frame, et al., J. Clinical Microbiology, Vol. 20, No. 2, pp. 162-166 (Auf. 1984) describe the identification and typing of herpes simplex virus using enzyme immunoassay and multiple unlabeled monoclonal antibodies.

Goldstein, et al., J. Infectious Diseases, Vol. 147, No. 51, pp. 829-837 (May 1983) developed a panel of unlabeled monoclonal antibodies reactive with either herpes simplex virus-1 or herpes simplex virus-2 which can be used reliably in immunofluorescent tests to serotype herpes simplex virus in culture and also for direct antigen detection and simultaneous typing of clinical specimens. These monoclonal antibodies were used separately and they were unlabeled.

Pereira, et al., Infection and Immunity, Vol. 35, No. 1, pp. 363-367 (Jan. 1982) describe use of panels of monoclonal antibodies for serotyping clinical isolates of herpes simplex virus with immunofluorescence, neutralization and immunoprecipitation tests.

Pereira, et al., Infection and Immunity, Vol. 29, No. 2, pp. 724-732 (Aug. 1980) describe the production of monoclonal antibodies useful to differentiate HSV-1 and HSV-2 and useful in immunofluorescence and neutralization tests.

In connection with immunoassays, the collection and transport of samples is also important. There are many references which discuss viral transport mediums. Of particular interest is B. F. Vestergaard's discussion of a detergent based transport medium in Methods of Enzymatic Analysis, 3d ed. Vol. X, Chapter 4.1 Herpes Simplex Virus, pp. 226-242 (1986).

## SUMMARY OF THE INVENTION

This invention is directed to solid phase immunoassays and kits for detecting or quantitating a substance having multiple epitopes utilizing principally IgG capture antibody and a cocktail of at least two labeled monoclonal antibodies as the detection system wherein each monoclonal antibody recognizes a distinct antigenic determinant.

The monclonals may be labeled with a component of a reporter system or they may be labeled with a member of a specific binding pair. When the monoclonals are labeled with a component of a reporter system, they may be detected and/or quantitated immediately.

However, when the monoclonals are labeled with a member of a specific binding pair they must be reacted with the second member of the binding pair which may be unlabeled or labeled. If the second member of the binding pair is labeled, then after the reaction ceases, detection and/or quantification may follow. If the second member of the binding pair is unlabeled then reaction of the bound pair with a component of a reporter system is needed in order to detect or quantify the substance suspected to be present.

## BRIEF DESCRIPTION OF THE DRAWINGS

FIG. 1 is a graph showing that combinations of labeled detector antibodies for herpes simplex virus produced a stronger signal than any signal generated by a corresponding amount of any individual monoclonal antibody.

FIG. 2 shows increased sensitivity achieved by using a cocktail of multiple monoclonal antibodies as the detection system.

FIG. 3 shows combinations of labeled detector monoclonal antibodies for cytomegalovirus generating a stronger detector signal than any signal generated by and individual monoclonal antibody.

## DESCRIPTION OF THE INVENTION

This invention is directed to an immunoassay for detecting or quantitating a substance having multiple epitopes comprising:

a) contacting a support with the sample suspected to contain said substance wherein the support has principally insolubilized IgG antibody attached thereto and said IgG antibody is specific for the suspected substance;

b) contacting the substance with a cocktail of at least two monoclonal antibodies wherein each monoclonal antibody recognizes a distinct antigenic determinant and wherein each monoclonal antibody is selected from the group consisting of (1) monoclonal antibodies labeled with a component of a reporter system and (2) monoclonal antibodies labeled with a first member of a specific binding pair;

c) reacting product of b(2) with the second member of the binding pair which may be unlabeled or labeled;

d) reacting product of step c with a component of a reporter system when the second member of the binding pair is unlabeled, and

e) detecting or quantitating the substance by reading the presence or absence of reporter.

According to the present invention, the immunoassay can be run as a forward sandwich assay in which the capture antibody is attached to a solid support followed by the addition of sample suspected to contain a substance having multiple epitopes. Once the reaction ceases, unreacted substance is washed away and a known quantity of the cocktail containing multiple labeled antibodies is added to the mixture.

Immunoassays according to this invention can also be run as simultaneous sandwich assays in which both the insolubilized capture antibody and cocktail of labeled antibodies and sample are added at the same time.

The capture antibody may be any immunoglobulin. However, the preferred embodiment of this invention utilizes principally IgG as the insolubilized capture antibody.

The support may be any one of a wide variety of supports, and as representative examples of suitable supports there may be mentioned: synthetic polymer supports, such as polystyrene, polypropylene, substituted polystyrene, e.g., aminated or carboxylated polystyrene, polyacrylamides, polyamides, polyvinylchloride, etc.; glass beads, agarose; etc. The supports may include reactive groups; e.g., carboxyl groups etc. to permit direct linking of the substance to the support.

In a preferred embodiment, the support consists of polystyrene microtiter plate wells.

According to the present invention, the substance which is being detected and/or quantitated is characterized by having multiple epitopes. By way of illustration only, the following substances can be mentioned as falling within the scope of this invention: polypeptides and proteins, polysaccharides, nucleic acids, and combinations thereof. There can also be mentioned bacteria, viruses, chromosomes, genes, mitochondria, nuclei, cell membranes, and the like. Exemplary proteins are nucleoproteins, glycoproteins, scleroproteins, proteoglycans, mucoproteins, histones, albumins, insulin, pepsin and the like. Furthermore, there can also be mentioned blood clotting factors such as fibrinogen, thrombin etc., as well as, peptide and protein hormones such as glucagon, follicle-stimulating hormone, luteinizing hormone, etc. Exemplary viruses include adenoviruses; herpes viruses such as herpes simplex, varicella zoster, herpes virus B, cytomegalovirus, Epstein-Barr virus, human herpes virus type 6; pox viruses; picornaviruses; orthomyxoviruses; paramyxoviruses; coronaviruses; rhabdoviruses; togaviruses; bunyaviruses; arenaviruses; rubella virus; arboviruses; reoviruses; hepatitis; retroviruses; oncogenic viruses and the like.

Techniques for preparing the monoclonal antibodies of the present invention are well known and have been cited in a wide variety of publications the following of which are incorporated by reference: Kohler and Milstein, Nature, 256: 495-497 (1975), Pereira, et al., Infection and Immunity, Vol. 29, No. 2, pp. 724-732 (Aug. 1980), Oi, et al., Immunoglobulin producing hybrid cell lines, pp. 351-371 in B. Mishell and S. Schiigi (ed.), Selected methods in cellular immunology, W. H. Freeman Co., San Francisco (1980) and Galfre et al., Preparation of Monoclonal Antibodies: Strategies and Procedures, Methods in Enzymology 73, pp. 1-46 (1981). These techniques will not be further described herein.

Depending upon the support selected the capture antibody may be either polyclonal or monoclonal. Attachment to the solid support, whether direct or indirect, covalent or non-covalent, may be achieved using well-known techniques.

In accordance with this invention all of the monoclonal antibodies are labeled using conventional techniques. They be labeled with a component of a reporter system or with a member of a specific binding pair.

Specific binding pairs may be of the immune or non-immune type. Immune specific binding pairs are exemplified by antigen-antibody systems or hapten/anti-hapten systems. There can be mentioned fluorescein/anti-fluorescein, dinitrophenyl/anti-dinitrophenyl, biotin/anti-biotin, peptide/anti-peptide and the like. The antibody member of the specific binding pair may be produced by customary methods familiar to those skilled in the art. Such methods involve immunizing an animal with the antigen member of the specific binding pair. If the antigen member of the specific binding pair is not immunogenic, i.e., a hapten, it may be covalently coupled to a carrier protein to render it immunogenic.

Non-immune binding pairs include systems wherein the two components share a natural affinity for each other but are not antibodies. Exemplary non-immune pairs are biotin-streptavidin, intrinisic factor-vitamin $B_{12}$, folic acid-folate binding protein and the like.

A variety of methods are available to covalently label monoclonal antibodies with members of specific binding pairs. Methods are selected based upon the nature of the member of the specific binding pair, the type of linkage desired, and the tolerance of the antibody to various conjugation chemistries. Biotin may be covalently coupled to monoclonal antibodies by utilizing commercially available active derivatives. Some of these are biotin-N-hydroxysuccinimide which binds to amine groups on proteins; biotin hydrazide which binds to carbohydrate moieties, aldehydes and carboxyl groups via a carbodiimide coupling; and biotin maleimide and iodoacetyl biotin which bind to sulfhydryl groups. Fluorescein can be coupled to protein amine groups using fluorescein isothiocyanate. Dinitrophenyl groups can be coupled to protein amine groups using 2,4-dinitrobenzene sulfonate or 2,4-dinitrofluorobenzene. Other standard methods of conjugation may be employed to couple monoclonal antibodies to a member of a specific binding pair including dialdehyde, carbodiimide coupling, homofunctional crosslinking, and heterobifunctional crosslinking. Carbodiimide coupling is an effective method of coupling carboxyl groups on one substance to amine groups on another. Carbodiimide coupling is facilitated by using the commercially available reagent, 1-ethyl-3-(dimethylaminpropyl)-carbodiimide (EDAC).

Homobifunctional crosslinkers, including the bifunctional imidoesters and bifunctional N-hydroxysuccinimide esters, are commercially available and are employed for coupling amine groups on one substance to amine groups on another. Heterobifunctional crosslinkers are reagents which possess different functional groups. The most common commercially available heterobifunctional crosslinkers have an amine reactive N-hydroxysuccinimide ester as one functional group, and a sulfhydryl reactive group as the second functional group. The most common sulfhydryl reactive groups are maleimides, pyridyl disulfides and active halogens. One of the functional groups may be a photoactive aryl nitrene, which upon irradiation reacts with a variety of groups. The preferred mode according to this inventions is conjugating monoclonal antibodies with biotin via an N-hydroxysuccinimide ester.

In order to facilitate signal detection, either the monoclonal antibodies or a member of a specific binding pair is labeled with a component of a reporting system. The term reporting system refers to the reporter selected and any means of linking the reporter to the monoclonal antibody or to a component of a specific binding pair. Thus, a reporter may be linked directly or indirectly, covalently or non-covalently to the monoclonal antibodies or to a member of a specific binding pair. Reporters may be radioactive isotopes, enzymes, fluorogenic, chemiluminescent or electrochemical materials. Two commonly used radioisotopes are $^{125}I$ and $^{3}H$. Standard radioactive isotopic labeling procedures include the chloramine T, lactoperoxidase and Bolton-Hunter methods for $^{125}I$ and reductive methylation for $^{3}H$.

Enzymes are also used as reporters for immunoassays. These include, but are not limited to, horseradish peroxidase, alkaline phosphatase, $\beta$-galactosidase, glucose oxidase, luciferase, $\beta$-lactamase, urease and lysozyme. Labeling with enzymes is facilitated by using dialdehyde, carbodiimide coupling, homobifunctional crosslinkers and heterobifunctional crosslinkers as described above for labeling monoclonal antibodies with members of specific binding pairs. The labeling method chosen depends on the functional groups available on the enzyme and the material to be labeled, and the tolerance of both to the conjugation conditions. The labeling method used in the present invention may be one of, but not limited to, any conventional methods currently employed including those described by Engvall and Pearlmann, Immunochemistry 8, 871 (1971), Avrameas and Ternynck, Immunochemistry 8, 1175 (1971), Ishikawa et al., J. Immunoassay 4(3):209-327 (1983) and Jablonski, Anal. Biochem. 148:199 (1985). Labeling may be accomplished by indirect methods such as using spacers or other members of specific binding pairs. An example of this is the detection of biotinylated antibodies with unlabeled streptavidin and biotinylated enzyme, with the unlabeled streptavidin and biotinylated enzyme being added either sequentially or simultaneously. Detection of enzyme activity may be facilitated by measuring chromogenic, fluorogenic, chemiluminescent or electrochemical changes by commonly known methods.

Reporters may be fluorogenic or chemiluminescent in nature. In addition, reporters may be detectable by electrochemical means. Some methods of labeling with these reporters are described above. In a preferred embodiment, the enzyme, horseradish peroxidase is used as the reporter and o-phenylenediamine/$H_2O_2$ is used as the substrate for chromogenic detection.

The monoclonal antibodies useful in the present invention may be of the IgG type as well as other immunoglobulins.

Moreover, these monoclonals may be directed to a multitude of antigenic determinants depending upon the target. By way of illustration only there can be mentioned antigenic determinants associated with nucleocapsid proteins, glycoproteins or viral envelopes.

In a preferred embodiment for virus detection, this invention also employs a collection and transport system specifically for the transport of samples for testing by immunoassay according to the invention.

The collection system utilizes any appropriate means of collecting a virus and the transport system utilizes a tube containing buffered detergent solution. After sample collection, the specimen is immersed in the buffered detergent. Direct immersion lyses the virus in the sample thus making more antigen accessible for testing and stablilizes the antigen during transport. The solution also contains a carrier protein which minimizes nonimmunologic binding of the proteins in the sample.

The following detergents can be mentioned as falling within the scope of the invention and should not be construed as a limitation: nonionic detergents such as polyoxyethylene ethers and alkylphenoxypolyethoxy compounds; ionic detergents such as alkyl/aryl sulfonates/sulfates; zwitterionic detergents; bile salts and derivatives thereof such as deoxycholic acid and alkyl saccharides such as octyl glucopyranoside and dodecyl maltoside.

Exemplary carrier proteins include but are not limited to serum, albumin, gelatin and casein.

Any buffer compatible with the assay system can be used to practice the invention.

In a most preferred embodiment, the buffer is phosphate buffered saline, the detergent is NONIDET P 40 (NP 40), and the carrier protein is bovine serum albumin.

In a further aspect the invention provides kits of reagents for carrying out the assays of the invention. Thus, for example, a kit according to the present invention may comprise (a) a support containing principally IgG antibodies, (b) a quantity of at least two monoclonal antibodies, wherein each monoclonal antibody recognizes a distinct antigenic epitope, and wherein each monoclonal antibody is selected from the group consisting of (1) monoclonal antibodies labeled with a reporter and (2) monoclonal antibodies labeled with a first member of a specific binding pair; (c) a quantity of the second member of the specific binding pair which may be labeled or unlabeled when (b) (2) is used: and (d) a quantity of a component of a reporter system when the second member of the binding pair is unlabeled.

The most preferred embodiment of the kits of reagents comprises all of the components (a)-(d) recited above and, in addition, a fifth component (e) which is a collection and transport system consisting of one or more plastic shafted cotton swabs and a tube containing a buffered detergent solution comprising buffer, detergent and a carrier protein.

The method of the present invention has a number of significant advantages such as increased sensitivity and specificity as evidenced by the data presented in Tables 1, 2 and 3.

Tables 1 and 2 present clinical data obtained by using the kit as referred to in Example 3. These clinical data demonstrate the high sensitivity and specificity achieved by using an immunoassay performed in accordance with the invention.

TABLE 1

Clinical Results Comparing Detection of HSV by Culture to Detection by an Immunoassay Performed According to the Invention

| STUDY | N | SENSITIVITY (%) (e) | SPECIFICITY (%) (f) | PPV(c) | NPV(d) |
|---|---|---|---|---|---|
| A | 337 | 24/26 (92) | 311/311(a) (100) | 100% | 99.4% |
| B | 56 | 15/15 (100) | 41/41(b) (100) | 100% | 100% |
| C | 82 | 38/40 (95) | 42/42(b) (100) | 100% | 95.4% |
| Total | 475 | 77/81 (95.1) | 394/394 (100) | 100% | 99.0% |

TABLE 1 (continued)

Notes:

a. Seven specimens were not included in this calculation. These specimens were cytopathic effect (CPE) negative and ELISA (enzyme linked immunosorbent assay) positive. The ELISA values were greater than 0.05 OD units above the mean of the negative controls (range 0.01 to >3.0 OD units above the mean of the negative controls and therefore are clearly antigen positive. All of these samples were tested with an antibody blocking confirmation test. Each sample was completely (>90%) inhibited by the addition of either rabbit or human anti-herpes simplex virus serum. The anti-herpes simplex virus serum was added at a concentration shown to be non-inhibitory in the assay. These findings indicate that the ELISA results were caused by the presence of herpes simplex virus antigen and were not non-specific positive reactions.

b. One specimen was not included in this calculation for the same reasons given in footnote a.

c. Positive Predictive Value (PPV) = True Positive/(True Positives + False Positives)

d. Negative Predictive Value (NPV) = True Negatives/(True Negatives + False Negatives)

e. Sensitivity = % of culture positive samples that tested positive by ELISA.

f. Specificity = % of culture negative samples that tested negative by ELISA.

Table 2 presents comparative data demonstrating the superiority of the instant invention over comparable methods. Table 3 compares results obtained using the instant invention against data set forth in the product inserts of the Ortho™ HSV ELISA and the Fairleigh Dickinson HSV ELISA. The Ortho test referred to in Tables 2 and 3 is a forward sandwich assay using polyclonal detection. The Fairleigh Dickinson test discussed in Table 3 is a simultaneous sandwich assay using polyclonal detection. As with Table 1, the data for the invention were obtained by using the kit as referred to in Example 3.

TABLE 2

| Comparison of Immunoassay Performed According to the Invention Against an Ortho Test | | | | | |
|---|---|---|---|---|---|
| STUDY | TEST (b) | SENSITIVITY (%) (c) | SPECIFICITY (%) (d) | PPV (%) | NPV (%) |
| A | INVENTION | 20/20 (100) | 233/233(a) (100) | 100 | 100 |
| | ORTHO | 9/20 (45) | 233/233 (100) | 100 | 86 |
| B | INVENTION | 15/15 (100) | 41/41(a) (100) | 100 | 100 |
| | ORTHO | 3/15 (20) | 42/42 (100) | 100 | 77 |
| Notes: | | | | | |

a. One ELISA " + ", tissue culture "-", confirmation test " + ", sample was not included in this data.
b. Ortho test was run as per manufacturer's directives.
c. Sensitivity = % of culture positive samples that tested positive by ELISA.
d. Specificity = % of culture negative samples that tested negative by ELISA.

TABLE 3

| COMPARISON OF TESTS FOR DETECTION OF HERPES SIMPLEX VIRUS | | | | |
|---|---|---|---|---|
| | INVENTION | ORTHO(a) | FAIRLEIGH(b) DICKINSON | CULTURE |
| SENSITIVITY | 95% | 55% | 61% | 100% |
| SPECIFICITY | 100% | 99% | 92% | 100% |
| SPECIMEN COLLECTION MEDIUM | HERPTRAN(TM)(h) | VTM(c) | VTM(c) | VTM(c) |
| TEST TIME | 4 hrs | 4.5 hrs | 1 hr | 1-7 days |
| TIME TO POSITIVE | 4 hrs | 4.5 hrs | 1 hr | 1-7 days |
| METHOD | ELISA | ELISA | ELISA | CULTURE |
| LABEL | SA-HRP(d)-OPD | HRP-OPD(e) | HRP-ABTS(f) | CPE(g) |
| Notes: | | | | |

(a) Product Insert, OrthoTM HSV Antigen ELISA Test, Ortho Diagnostic Systems, Inc., Raritan, NJ.
(b) Product Insert, ELISA for Herpes Simplex Virus, Fairleigh Dickinson Labs. Inc., Abilene, TX.
(c) VTM represents viral transport medium.
(d) SA-HRP-OPD is streptavidin-horseradish peroxidase-orthophenylene-diamine.
(e) HRP-OPD is horseradish peroxidase-orthophenylenediamine.
(f) HRP-ABTS is horseradish peroxidase-2,2′-Azino-bis-(3-ethylbenzthiazolinesulfonic Acid).
(g) CPE is cytopathic effect.
(h) HERPTRANTM specimen collection system is discussed in Example 3.

The following examples are intended to illustrate the invention and should not be construed as limitations thereon.

## EXAMPLE 1

## Cocktails of Labeled Monoclonal Antibodies Identified as 55306, 55307 and 55308

### Monoclonal Antibody Preparation

The monoclonal antibodies were prepared according to the methods of L. Pereira, et al., as described in Infection and Immunity, Vol. 29. No. 2, pp. 724-732 (Aug. 1980) and Oi, et al. as described in Immunoglobulin Producing Hybrid cell lines, p. 351-371, in B. Mishell and S. Schiigi (ed.), Selected Methods in Cellular Immunology, W. H. Freeman Co., San Francisco.

Epitope specificities were determined by competing unlabeled antibody with biotin labeled antibody for solid phase antigen. When an unlabeled antibody did not inhibit the binding of a labeled antibody, it was determined that the antibodies were directed against different epitopes.

Table 4 as discussed in Example 2 sets forth the correlation between the monoclonal antibodies and their particular specificities. Thus, monoclonals identified as 55306-55308 in this example have the same specificities set forth in Table 4 but a different concentration was used. In this example, each detector monoclonal antibody was used at a 1/1000 dilution.

Monoclonal antibodies were purified from ascites fluid by salt fractionation with 40% saturated ammonium sulfate and gel filtration on Sephacryl S-300 (Pharmacia, Inc., Piscataway, NJ). The monoclonal antibodies were biotinylated as described below. To reiterate, each detector monclonal antibody was used at a 1/1000 dilution.

### Antibody biotinylation

Antibody solutions were dialyzed against 0.1M $NaHCO_3$ buffer, pH 8.4, and adjusted to a concentration of 1 mg in 1 ml. A 50 fold molar excess of a biotin-N-hydroxysuccinimide ester (commercially available from a variety of sources, including Sigma Chemical Co., St. Louis, MO and Calbiochem, San Diego, CA) in 0.1 ml dimethylsulfoxide was added and allowed to react for 2 hours at room temperature, at which time 0.1 ml of 2M tris-HCl pH 8.0 was added. After 15 minutes, 1 ml of 1% bovine serum albumin (Sigma) in phosphate buffered saline (PBS) pH 7.4 was added and the solution dialyzed against PBS. The biotinylated antibodies were stored at 4° C.

### Assay

Polystyrene microtiter plate wells were coated with 0.1 ml polyclonal antibody reactive against both HSV-1 and HSV-2 (Dako Corp., Santa Barbara, CA) diluted in 0.1 M carbonate buffer, pH 9.6, and incubated overnight at 4° C. The wells were washed with PBS containing 0.05% Tween 20 (PBS-T), and blocked with 2% bovine serum albumin (BSA) in carbonate buffer for 1 hour at room temperature. The wells were washed with PBS-T and 0.1 ml of HSV-1 antigen diluted in 1% BSA-PBS-T was added and incubated for 1 hour at 37° C. The wells were washed with PBS-T and 0.1 ml of biotinylated detector antibody, diluted in 1% BSA-PBS-T was added and incubated for 30 minutes at room temperature. Combinations of two biotinylated monoclonal antibodies were prepared by mixing one part of each of the detectors (i.e., each antibody contributed one half of the total mass of detector antibody). A combination of the three antibodies was prepared by mixing one part of each detector (i.e., each contributed one third of the total mass). The wells were washed with PBS-T and 0.1 ml of a streptavidin-horseradish peroxidase conjugate (Bethesda Research Laboratories, Gaithersburg, MD), diluted in 1% BSA-PBS-T, was added and incubated for 15 minutes at room temperature. The wells were washed with PBS-T and 0.1 ml of peroxidase substrate (0.05% o-phenylenediamine, 0.01% $H_2O_2$ in 0.1 M citrate buffer, pH 5.0) was added. Following color development, the reaction was stopped with $H_2SO_4$, and the absorbance at 490 nm determined.

## Results

The optical densities (O.D.) reported were determined by subtracting the signal of a blank well (with no antigen present) from the signal generated by the sample well. The intensity of the signal produced by each of the three detectors (Fig. 1) was noticeably different, with 55306 giving the greatest signal and 55307, the poorest. The combination of any two or three detector antibodies generated a greater signal than that of any individual antibody having a mass corresponding to the total mass of any combination. This clearly demonstrates the synergistic effect of combining detector antibodies.

## EXAMPLE 2

### Performance of a Single Monoclonal Antibody Detector vs. a Monoclonal Detection Cocktail at the Optimal Concentration of Each Monoclonal Antibody Preparation

Nine monoclonal antibodies identified as (55306-55314) specific for herpes simplex virus glycoproteins were prepared, purified and biotinylated as described above. Optimal concentrations of each detector monoclonal antibody were determined by a dilution series of detector, and selecting the concentration giving maximum signal to background.

Table 4 sets forth the specificity and concentration of each monoclonal antibody used in the three Examples 2 and 3 and the specificity of monoclonal antibodies used in Example 1.

Table 4

| HSV Antibodies | | |
|---|---|---|
| Ab | Specificity | Concentration |
| 55306 | HSV 1 and 2 gD | 1/1600 |
| 55307 | HSV 1 and 2 gD | 1/800 |
| 55308 | HSV 1 and 2 gD | 1/1600 |
| 55309 | HSV 1 gC | 1/800 |
| 55310 | HSV 1 gB | 1/1600 |
| 55311 | HSV 2 gG | 1/800 |
| 55312 | HSV 2 gG | 1/1600 |
| 55313 | HSV 2 gB | 1/1600 |
| 55314 | HSV 2 gG | 1/1600 |

## Assay

Polystyrene microtiter plate wells were coated with 0.1 ml polyclonal antibody reactive against both HSV-1 and HSV-2 (Dako Corp., Santa Barbara, CA) diluted in 0.1 M carbonate buffer, pH 9.6, and incubated overnight at 4° C. The wells were washed with PBS containing 0.05% Tween 20 (PBS-T), and blocked with 2% bovine serum albumin (BSA) in carbonate buffer for 1 hour at room temperature. The wells were washed with PBS-T and 0.1 ml of HSV-1 antigen diluted in 1% BSA-PBS-T was added and incubated for 2 hours at 37° C. The wells were washed with PBS-T and 0.1 ml of biotinylated detector antibody, or cocktail of antibodies, diluted in 50% normal rabbit serum-0.1% sodium caseinate-PBS was added, and incubated for 30 minutes at room temperature. The wells were washed with PBS-T and 0.1 ml of a streptavidin-horseradish peroxidase conjugate, diluted in 1% BSA-PBS-T, was added and incubated for 15 minutes at room temperature. The wells were washed with PBS-T and 0.1 ml of peroxidase substrate (0.05% o-phenylenediamine, 0.01% $H_2O_2$ in 0.1M citrate buffer, pH 5.0) was added. Following color development, the reaction was stopped with $H_2SO_4$, and the absorbance at 490 nm determined.

## Results

Figure 2 shows the comparison of a single monoclonal detector compared to a cocktail of nine monoclonal antibodies. The cutoffs were determined by adding 0.05 to the blank readings. Use of a monoclonal antibody detector cocktail results in improved assay sensitivity, as well as an approximately five fold lower detection limit of viral antigen.

## EXAMPLE 3

### A Kit of the Detection of Herpes Simplex Virus Antigen Utilizing Multiple Monoclonal Antibody Detection

### Kit Composition

(a) a solid support (polystyrene microtiter plate) coated with antibody reactive against herpes simplex virus-1 and herpes simplex virus-2. (b) a quantity of multiple biotinylated monoclonal antibodies specific for herpes simplex virus-1 and herpes simplex virus-2. (c) enzyme labeled streptavidin (streptavidin-horseradish peroxidase). (d) a substrate for determining enzyme activity (o-phenylenediamine/$H_2O_2$). (e) collection and transport system.

### Assay

As described in Example 2 for the invention. In addition, some samples were tested with the Ortho™ HSV Antigen ELISA Test (Ortho Diagnostic Systems Inc., Raritan, NJ).

### Clinical Specimens

Duplicate specimens were collected with cotton swabs, one placed into medium for viral culture, and the second placed into Du Pont Herptran™ medium for ELISA. These specimens were collected from obstetric patients or patients suspected to have herpes whether or not these patients manifested visible signs of infection. The Herptran™ medium consists of phosphate buffered saline, NONIDET P 40 (NP 40) and bovine serum albumin. Specimens for culture were either cultured immediately or stored at -70°C. Specimens for ELISA testing were stored at 4°C until tested.

## Results

To date, 475 specimens have been tested (Table 1), with a clinical sensitivity of 95% (77/81) and specificity of 100% (394/394) as compared to culture. The clinical performance is compared to the commercially available Ortho test in Table 2. The data in Tables 1 and 2 indicate that the performance of the assay is superior to any herpes simplex virus enzyme immunoassay thus far commercialized or reported in the literature.

## EXAMPLE 4

## Combining Monoclonal Antibodies to Cytomegalovirus (CMV)

### Preparation of Monoclonal Antibodies

Three anti-CMV monoclonal antibodies (designated as A, B, and C) were prepared, purified and biotinylated as described in Example 1. Optimal concentrations were determined using a dilution series of detector antibody, selecting the concentration giving maximum signal to background.

Table 5 sets forth the specificity and concentration of each monoclonal antibody discussed in Example 4.

Table 5

| Ab | Specificity | Concentration |
|---|---|---|
| A | glycoprotein A | 1/733 |
| B | glycoprotein A | 1/1467 |
| C | glycoprotein A | 1/2933 |

### Assay

Polystyrene microtiter plate wells were coated with a polyclonal rabbit anti-CMV antibody diluted in 0.1 M carbonate buffer pH 9.6 overnight at 4°C. The wells were washed with PBS-T and blocked with 2% BSA in carbonate buffer for 2 hours at 37°C. The wells were washed with PBS-T. CMV antigen (diluted in Du Pont HerptranTM) was added and incubated for 2 hours at 37°C. The wells were washed with PBS-T and detector antibody and combinations of detector antibodies diluted to their optimal concentrations in 1% BSA-PBS-T were added and incubated for 1 hour at room temperature. The wells were washed with PBS-T and a streptavidin-horseradish peroxidase conjugate, diluted in 1% BSA-PBS-T was added and incubated for 15 minutes at room temperature. The wells were washed with PBS-T an a peroxidase substrate (0.05% o-phenylenediamine, 0.01% $H_2O_2$, in citrate buffer pH 5.0) was added. After color development, the reaction was stopped with $H_2SO_4$, and the absorbance was determined at 490 nm.

### Results

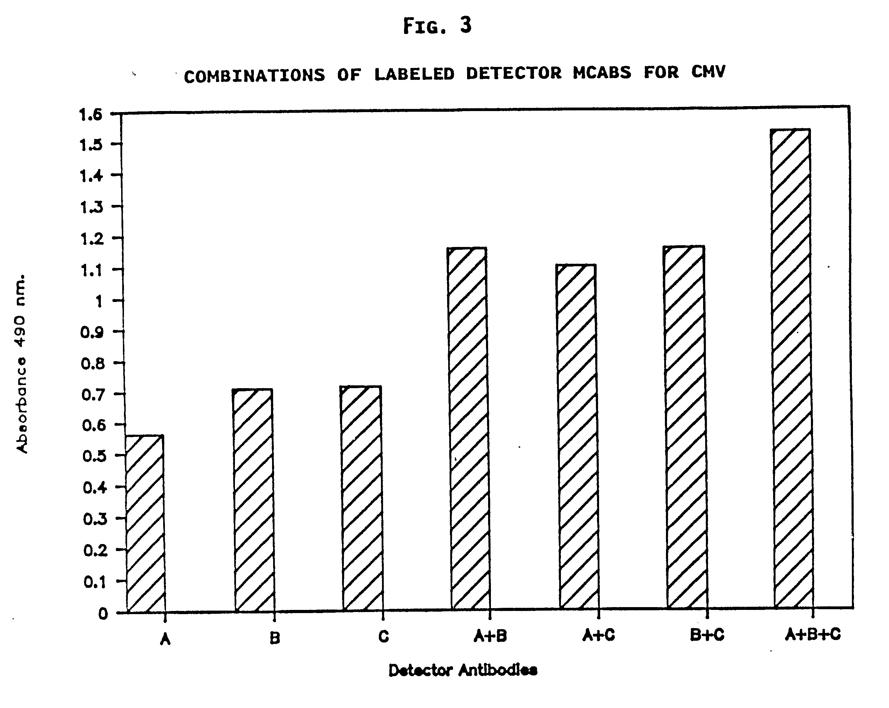

Figure 3 illustrates the improved performance resulting from the utilization of combinations of monoclonal antibodies as compared to single monoclonal antibody detectors for CMV.

## Claims

1. An immunoassay for detecting or quantitating a substance having multiple epitopes comprising:

a) contacting a support with the sample suspected to contain said substance wherein the support has principally insolubilized IgG antibody attached thereto and said IgG antibody is specific for the suspected substance;

b) contacting the substance with a cocktail of at least two monoclonal antibodies wherein each monoclonal antibody recognizes a distinct antigenic determinant and wherein each monoclonal antibody is selected from the group consisting of (1) monoclonal antibodies labeled with a component of a reporter system, and (2) monoclonal antibodies labeled with a first member of a specific binding pair;

c) reacting product of b(2) with the second member of the binding pair which may be unlabeled or labeled;

d) reacting product of step c with a component of a reporter system when the second member of the binding pair is unlabeled, and

e) detecting or quantitating the substance by reading the presence or absence of reporter.

2. An immunoassay according to claim 1 wherein the substance is selected from the group consisting of polypeptides, proteins, polysaccharides, nucleic acids, blood clotting factors, hormones, bacteria and viruses.

3. An immunoassay according to claim 1 wherein the monoclonal antibodies are of the IgG type.

4. An immunoassay according to claim 1 wherein said immunoassay is conducted as a sequential forward immunoassay.

5. An immunoassay according to claim 1 wherein said immunoassay is conducted as a simultaneous immunoassay.

6. An immunoassay according to claim 1 wherein the monoclonal antibodies are labeled with one component of an immune or non-immune specific binding pair.

7. An immunoassay according to claim 1 wherein the monoclonal antibodies are labeled with biotin.

8. An immunoassay according to claim 7 wherein the biotinylated monoclonal antibodies are contacted with streptavidin-horseradish peroxidase.

9. An immunoassay according to claim 8 wherein the product of claim 8 is reacted with orthophenylenediamine as enzyme substrate.

10. An immunoassay according to claim 1 wherein the reporter is selected from the group consisting of radioactive isotopes, enzymes, fluorogenic, chemiluminescent and electrochemical materials.

11. An immunoassay according to claim 10 wherein the reporter is coupled directly or indirectly, covalently or non-covalently.

12. An immunoassay for detecting or quantitating a virus having multiple epitopes comprising:

a) contacting a support with the sample suspected to contain said substance wherein the support has principally insolubilized IgG antibody attached thereto and said IgG antibody is specific for the suspected substance;

b) contacting the substance with a cocktail of at least two monoclonal antibodies wherein each monoclonal antibody recognizes a distinct antigenic determinant and wherein each monoclonal antibody is selected from the group consisting of (1) monoclonal antibodies labeled with a component of a reporter system, and (2) monoclonal antibodies labeled with a first member of a specific binding pair;

c) reacting product of b(2) with the second member of the binding pair which may be unlabeled or labeled;

d) reacting product of step c with a component of a reporter system when the second member of the binding pair is unlabeled, and

e) detecting or quantitating the substance by reading the presence or absence of reporter.

13. An immunoassay according to claim 12 wherein the virus is selected from the group consisting of adenoviruses, herpes simplex, varicella zoster, herpes virus B, cytomegalovirus, Epstein-Barr virus, pox viruses, picornaviruses, orthomyxoviruses, paramyxoviruses, coronaviruses, rhabdoviruses, togaviruses, bunyaviruses, arenaviruses, rubella virus, arboviruses, reoviruses, hepatitis, retroviruses and oncogenic viruses.

14. An immunoassay according to claim 12 wherein the monoclonal antibodies are of the IgG type.

15. An immunoassay according to claim 12 wherein said immunoassay is conducted as a sequential forward immunoassay.

16. An immunoassay according to claim 12 wherein said immunoassay is conducted as a simultaneous immunoassay.

17. An immunoassay according to claim 12 wherein the monoclonal antibodies are labeled with biotin.

18. An immunoassay according to claim 17 wherein the biotinylated monoclonal antibodies are contacted with streptavidin-horseradish peroxidase.

19. An immunoassay according to claim 18 wherein the product of claim 18 is reacted with orthophenylenediamine as the enzyme substrate.

20. An immunoassay according to claim 12 wherein the monoclonal antibodies are labeled with one component of an immune or non-immune specific binding pair.

21. An immunoassay according to claim 12 wherein the reporter is selected from the group consisting of radioactive isotopes, enzymes, fluorogenic, and chemiluminescent and electrochemical materials.

22. An immunoassay according to claim 21 wherein the reporter is coupled directly or indirectly, covalently or non-covalently.

23. An immunoassay according to claim 12 wherein the sample is transferred directly into a tube containing a buffered detergent solution comprising buffer, detergent and a carrier protein.

24. An immunoassay for detecting or quantitating a herpes virus having multiple epitopes comprising:

a) contacting a support with the sample suspected to contain said substance wherein the support has principally insolubilized IgG antibody attached thereto and said IgG antibody is specific for the suspected substance;

b) contacting the substance with a cocktail of at least two monoclonal antibodies wherein each monoclonal antibody recognizes a distinct antigenic determinant and wherein each monoclonal antibody is selected from the group consisting of (1) monoclonal antibodies labeled with a component of a reporter system, and (2) monoclonal antibodies labeled with a first member of a specific binding pair;

c) reacting product of b(2) with the second member of the binding pair which may be unlabeled or labeled;

d) reacting product of step c with a component of a reporter system when the second member of the binding pair is unlabeled, and

e) detecting or quantitating the substance by reading the presence or absence of reporter.

25. An immunoassay according to claim 24 wherein the herpes virus is selected from the group consisting of HSV-1, HSV-2, and cytomegalovirus.

26. An immunoassay according to claim 24 wherein the monoclonal antibodies are of the IgG type.

27. An immunoassay according to claim 24 wherein said immunoassay is conducted as a sequential forward immunoassay.

28. An immunoassay according to claim 24 wherein said immunoassay is conducted as a simultaneous immunoassay.

29. An immunoassay according to claim 24 wherein the monoclonal antibodies are labeled with one component of an immune or non-immune specific binding pair.

30. An immunoassay according to claim 24 wherein the monoclonal antibodies are labeled with biotin.

31. An immunoassay according to claim 30 wherein the biotinylated monoclonal antibodies are contacted with streptavidin-horseradish peroxidase.

32. An immunoassay according to claim 31 wherein the product of claim 31 is reacted with orthophenylenediamine as enzyme substrate.

33. An immunoassay according to claim 24 wherein the reporter is selected from the group consisting of radioactive isotopes, enzymes, fluorogenic, chemiluminescent and electrochemical materials.

34. An immunoassay according to claim 33 wherein the reporter is coupled directly or indirectly, covalently or non-covalently.

35. An immunoassay according to claim 24 wherein the sample is transferred directly into a tube containing a buffered detergent solution comprising buffer, detergent and a carrier protein.

36. A kit of reagents for carrying out an immunoassay for detecting or quantitating a substance having multiple epitopes which comprises:

a) a support containing principally IgG antibodies;

b) a quantity of at least two monoclonal antibodies wherein each monoclonal antibody recognizes a distinct antigenic epitope and wherein each monoclonal antibody is selected from the group consisting of (1) monoclonal antibodies labeled with a component of a reporter system and (2) monoclonal antibodies labeled with a first member of a specific binding pair:

c) a quantity of the second member of the specific binding pair which may be labeled or unlabeled when b(2) is used; and

d) a quantity of a component of a reporter system when the second member of the binding pair is unlabeled.

37. A kit according to claim 36 wherein the substance is selected from the group consisting of polypeptides, proteins, polysaccharides, nucleic acids, blood clotting factors, hormones, bacteria and viruses.

38. A kit according to claim 36 wherein the monoclonal antibodies are of the IgG type.

39. A kit according to claim 36 wherein the monoclonal antibodies are labeled with one component of an immune or non-immune specific binding pair.

40. A kit according to claim 36 wherein the reporter is selected from the group consisting of radioactive isotopes, enzymes, fluorogenic, chemiluminescent and electrochemical materials.

41. A kit according to claim 40 wherein the reporter is coupled indirectly, covalently or non-covalently.

42. A kit according to claim 36 wherein the monoclonal antibodies are labeled with biotin.

43. A kit according to claim 42 wherein the biotinylated monoclonal antibodies are contacted with streptavidin-horeseradish peroxidase.

44. A kit according to claim 43 wherein the product of claim 43 is reacted with orthophenylenediamine as enzyme substrate.

45. A kit of reagents for carrying out an immunoassay for detecting or quantitating a virus having multiple epitopes which comprises:

a) a support containing principally IgG antibodies;

b) a quantity of at least two monoclonal antibodies wherein each monoclonal antibody recognizes a distinct antigenic epitope and wherein each monoclonal antibody is selected from the group consisting of (1) monoclonal antibodies labeled with a component of a reporter system and (2) monoclonal antibodies labeled with a first member of a specific binding pair;

c) a quantity of the second member of the specific binding pair which may be labeled or unlabeled when b(2) is used;

d) a quantity of a component of a reporter system when the second member of the binding pair is unlabeled; and

e) a transport system consisting of a tube containing a buffered detergent solution comprising buffer, detergent and a carrier protein.

46. A kit according to claim 45 wherein the virus is selected from the group consisting of adenoviruses, herpes simplex, varicella zoster, herpes virus B, cytomegalovirus, Epstein-Barr virus, pox viruses, picornaviruses, orthomyxoviruses, paramyxoviruses, coronaviruses, rhabdoviruses, togaviruses, bunyaviruses, arenaviruses, rubella virus, arboviruses, reoviruses, hepatitis, retroviruses and oncogenic viruses.

47. A kit according to claim 45 wherein the monoclonal antibodies are of the IgG type.

48. A kit according to claim 45 wherein the monoclonal antibodies are labeled with one component of an immune or non-immune specific binding pair.

49. A kit according to claim 45 wherein the reporter is selected from the group consisting of radioactive isotopes, enzymes, fluorogenic, chemiluminescent and electrochemical materials.

50. A kit according to claim 49 wherein the reporter is coupled directly or indirectly, covalently or non-covalently.

51. A kit according to claim 45 wherein the monoclonal antibodies are labeled with biotin.

52. A kit according to claim 51 wherein the biotinylated monoclonal antibodies are contacted with streptavidin-horseradish peroxidase.

53. A kit according to claim 52 wherein the product of claim 52 is reacted with orthophenylenediamine as enzyme substrate.

54. A kit of reagents for carrying out an immunoassay for detecting or quantitating a herpes virus having multiple epitopes which comprises:

a) a support containing principally IgG antibodies;

b) a quantity of at least two monoclonal antibodies wherein each monoclonal antibody recognizes a distinct antigenic epitope and wherein each monoclonal antibody is selected from the group consisting of (1) monoclonal antibodies labeled with a component of a reporter system and (2) monoclonal antibodies labeled with a first member of a specific binding pair;

c) a quantity of the second member of the specific binding pair which may be labeled or unlabeled when b(2) is used;

d) a quantity of a component of a reporter system when the second member of the binding pair is unlabeled; and

e) a transport system consisting of a tube containing a buffered detergent solution comprising buffer, detergent and a carrier protein.

55. A kit according to claim 54 wherein the monoclonal antibodies are of the IgG type.

56. A kit according to claim 54 wherein the substance is a herpes virus selected from the group consisting of HSV-1, HSV-2 and cytomegalovirus.

57. A kit according to claim 54 wherein the monoclonal antibodies are labeled with one component of an immune or non-immune specific binding pair.

58. A kit according to claim 54 wherein the reporter is selected from the group consisting of radioactive isotopes, enzymes, fluorogenic, chemiluminescent and electrochemical materials.

59. A kit according to claim 58 wherein the reporter is coupled directly or indirectly, covalently or non-covalently.

60. A kit according to claim 54 wherein the monoclonal antibodies are labeled with biotin.

61. A kit according to claim 60 wherein the biotinylated antibodies are contacted with streptavidin-horseradish peroxidase.

62. A kit according to claim 61 wherein the product of claim 61 is reacted with orthophenylenediamine is enzyme substrate.

FIG. 1

COMBINATIONS OF LABELED DETECTOR
MONOCLONAL ANTIBODIES FOR HSV

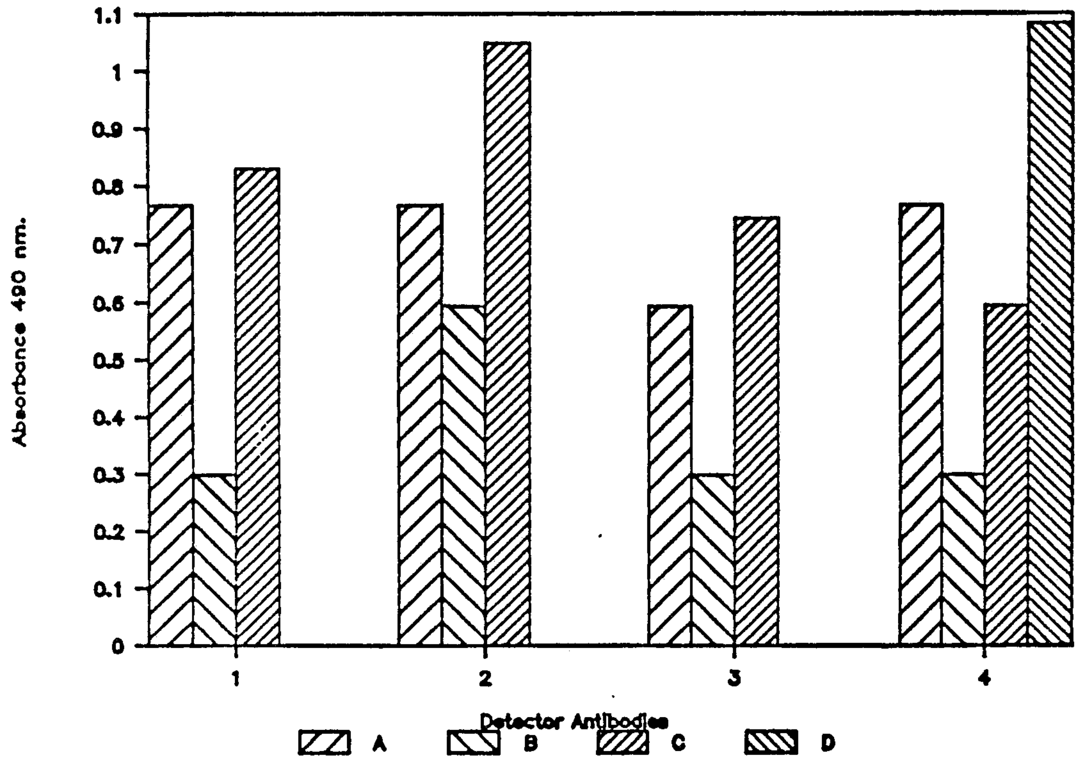


| Detector | Concentration | Detector | Concentration |
|---|---|---|---|
| 1A 55306 | 1/1000 | 3A 55307 | 1/1000 |
| 1B 55308 | 1/1000 | 3B 55308 | 1/1000 |
| 1C (55306 + 55308) | 1/1000 | 3C (55307 + 55308) | 1/1000 |
| 2A 55306 | 1/1000 | 4A 55306 | 1/1000 |
| 2B 55307 | 1/1000 | 4B 55308 | 1/1000 |
| 2C (55306 + 55307) | 1/1000 | 4C 55307 | 1/1000 |
| | | 4D (55306 + 55308 + 55307) | 1/1000 |

Fig. 2
HSV Detection

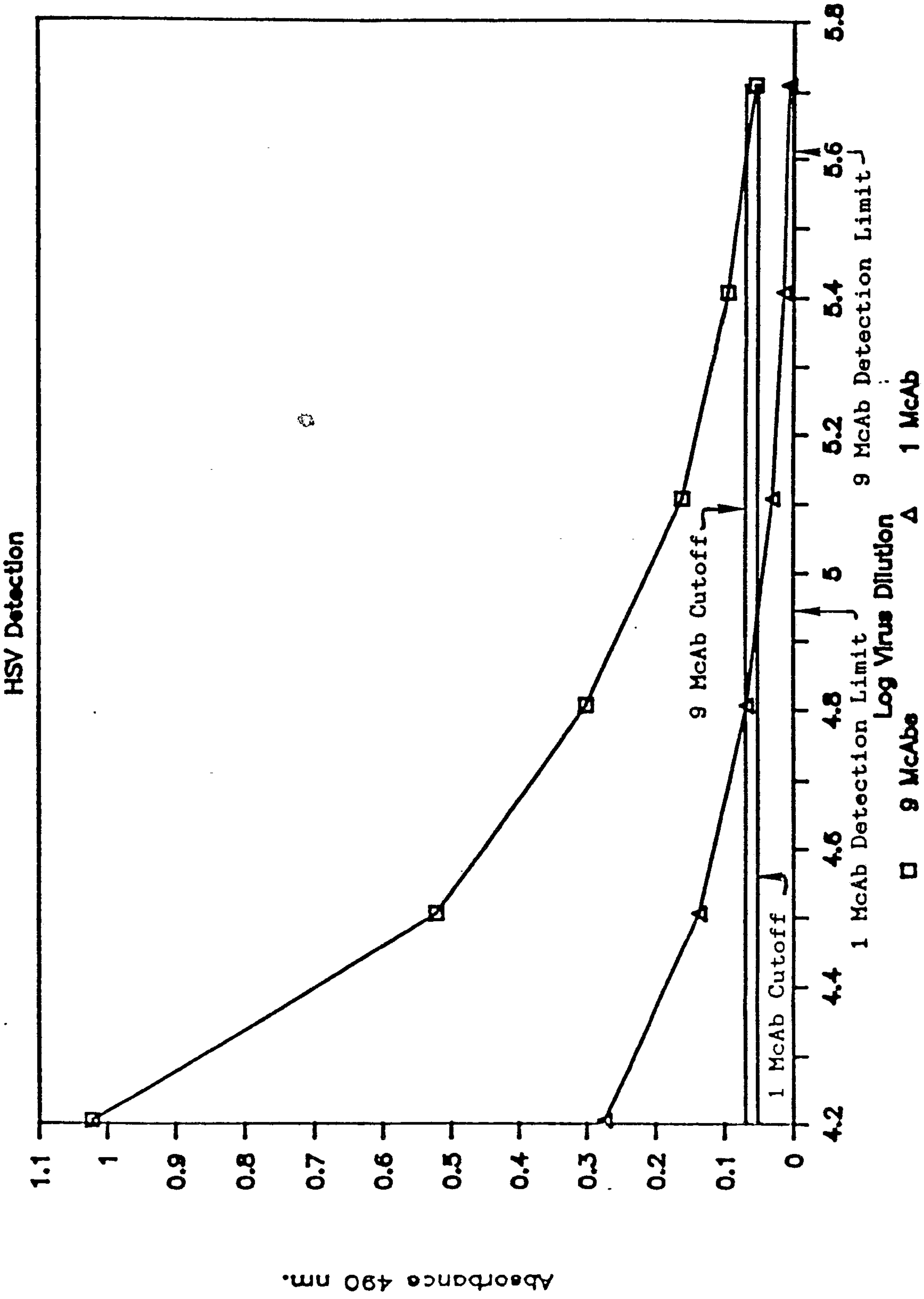

FIG. 3

COMBINATIONS OF LABELED DETECTOR MCABS FOR CMV